# EUROPEAN PATENT APPLICATION

(11) **EP 0 682 914 A1**
(43) Date of publication of application: **22.11.1995**
(21) Application number: 94830226.0
(22) Date of filing: 17.05.1994
(51) Int. Cl.: A61B 6/00, A61B 6/04

(54) **A table for diagnostic examinations**

(71) Applicant: C.A.T. DI CORSINI GIUSEPPE & C. S.p.A., I-40044 Pontecchio Marconi (Bologna) (IT)
(72) Inventor: Sitta, Stefano, I-40044 Pontecchio Marconi, Bologna (IT)
(74) Representative: Lanzoni, Luciano

(57) **Abstract**

A table for diagnostic examinations, of the type comprising a platform (2), anchored pivotably by way of restraint means (21) to a first horizontal axis (X) and rotatable thus through a plurality of positions compassing and including a first limit position (A) in which the platform (2) is disposed substantially vertical, rotated 90^{o} about the first horizontal axis (X) in a first direction (R) away from a horizontal reference plane (PO) occupied by the selfsame first axis (X) and disposed at a first fixed height (DX) above datum, and a second limit position (Z) in which the platform (2) is rotated away from the horizontal reference plane (PO) in a direction (R1) opposite to the first direction, characterized in that the restraint means (21) are associated with a first end (22) of the platform (2), at a point separated from the relative extremity by a distance (D2) no greater than the fixed height (DX) of the first horizontal axis (X) above datum, and in that it comprises actuating and positioning means (3) consisting in elements of selectively variable combined length, pivotably associated at a first end or stationary end (31) with a second horizontal axis (W) disposed parallel beneath and vertically offset relative to the first horizontal axis (X), and at a second end or moving end (32) with a third horizontal axis (J) passing through the platform (2) at the end opposite to the first end (22) occupied by the first horizontal axis (X), in such a way that an increase or a reduction in length of the actuating and positioning means (3), and consequently of the distance between centres of the second and third horizontal axes (W, J), will cause the platform (2) to assume a position coinciding with or between the first and second limit positions (A, Z).

## Description

The present invention relates to a table on which to conduct diagnostic examinations, in particular a table serving to support a patient, such as can be tilted to a variable angle.

The techniques currently adopted in carrying out a range of diagnostic tests, for example radiological examinations on the gastrointestinal tract, on the lungs or on the urogenital and nervous systems, are dependent at least in part on varying the position of the patient under examination; for this reason, the patient generally is extended upon and suitably secured to a special table substantially having the freedom to pivot about one or more horizontal axes, in such a way as will allow rotation about at least one of these axes to maximize the effectiveness of the test.

Reference may be made in this regard to EP 146 006 and EP 244 560, which relate to radiology equipment comprising a table designed for use in situations of the type mentioned above. The underside of the table in question is secured rigidly to a carriage element presenting the shape (or rather the volume) essentially of a cylindrical sector radiating from a horizontal axis, of which the arcuate surface is directed downwards. This same carriage element is supported from beneath, free to pivot about its own axis, and connected thus to a suitable actuating system or mechanism capable of generating movement whereby the patient can be arranged in any position ranging between an essentially upright stance with head uppermost, and a recumbent position in which the body is marginally inclined in relation to a horizontal plane and the head at a level slightly lower than that of the feet.

Whilst the patent solutions referred to above may differ one from another in minor details, such as the shape exhibited by certain of the sliding ways, both betray drawbacks deriving from the geometry of the carriage element located beneath the table: the dimensions of this element are such, in effect, by reason of the circumferential portion operating in association with the actuating system or mechanism, as to fill the entire area below the table proper and thus maximize the overall space occupied by the equipment.

Moreover, the association of the carriage element with the table has the effect of increasing the masses brought into play, a factor that dictates the need for an actuating system of considerable power and, at the same time, for floor anchorages of massive proportions. In addition, there is the requirement for particularly complex transmission components such as racks with curvilinear tooth profiles, which do little to simplify the mechanics of the actuating system.

Accordingly, the object of the present invention is to overcome the drawbacks outlined above.

The stated object is realized, according to the invention, in a table for diagnostic examinations comprising a pivotable platform, and, associated with the platform, linear actuating and positioning means of simple embodiment and variable length such as will allow stable positioning at or between two limit positions: a first in which the platform is disposed substantially vertical, rotated 90^{o} in a first direction about a horizontal axis away from a horizontal reference plane, and a second in which the platform is rotated in a direction opposite to the first direction and lies below the horizontal reference plane.

Advantageously, a reduced transverse dimension is achieved according to the present invention, thanks to the adoption of actuating means which do not project from the bearing structure of the table; moreover, additional advantage is afforded by the use of actuating means operating in a single plane and capable of holding a selected position without generating any oscillation whatever in the table once the desired angle has been assumed.

The invention will now be described in detail, by way of example, with the aid of the accompanying drawings, in which:
- fig 1 shows a possible embodiment of the table according to the invention, in a side elevation schematically indicating three typical operating positions;
- fig 2 shows the table according to the invention in a side elevation with certain parts omitted to reveal others;
- figs 3, 4 and 5 show a possible embodiment of actuating and positioning means according to the invention, seen in a side elevation with certain parts omitted to reveal others, respectively in a position of minimum extension, in an intermediate position and in a position of maximum extension.

With reference to the accompanying drawings, which illustrate one possible embodiment of a table for diagnostic examinations according to the invention, such a table 1 consists essentially in a platform 2 anchored rotatably to a horizontal axis and set in motion by actuating and positioning means 3 in such a way as to assume the angle most suitable for the particular examination to be carried out.

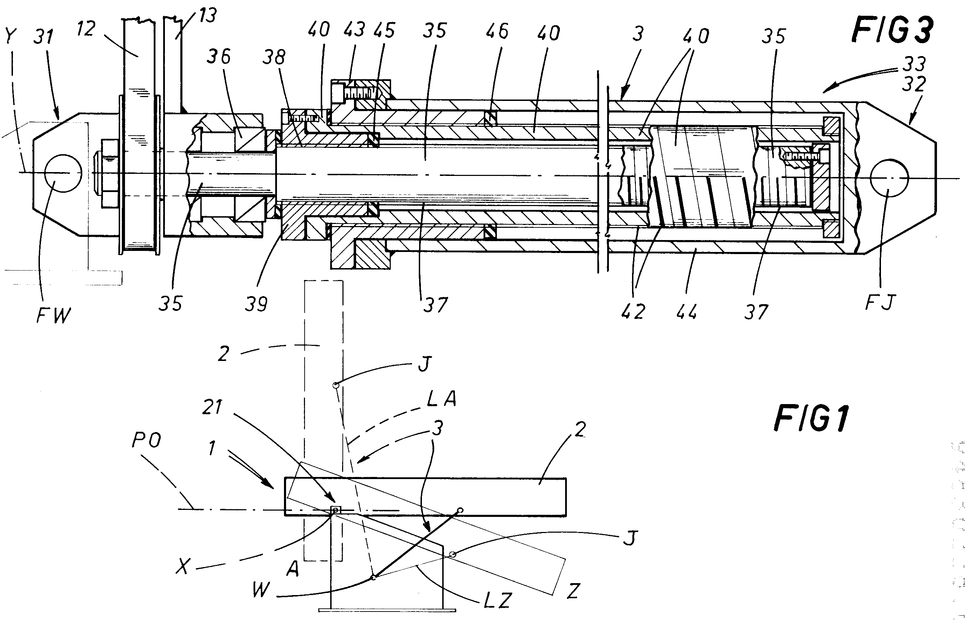

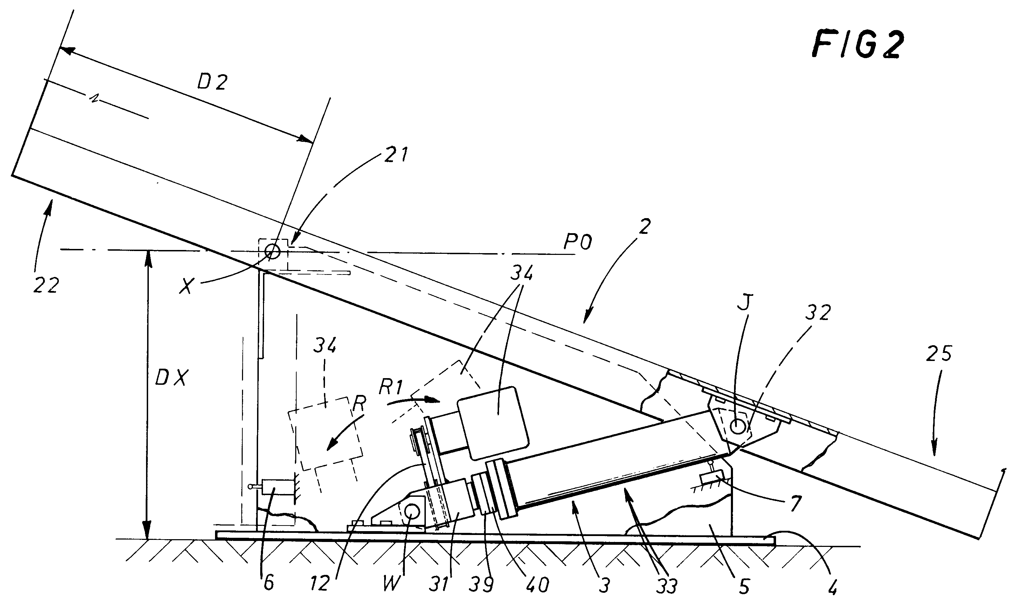

As discernible in particular from the schematic representations of fig 1 and fig 2, the platform 2 is associated by way of restraint means 21 with a first horizontal axis X and rotatable about this same axis through a plurality of stable positions between a first limit position A and a second limit position Z.

In the first such limit position A, the platform 2 is disposed substantially vertical, or in effect, rotated about the first axis X through 90^{o} in a first direction R (toward the left, as viewed in the example illustrated) away from a horizontal reference plane PO which is occupied by the first axis X and disposed at a fixed height above datum, denoted DX.

In the second limit position Z, the platform 2 is again rotated from the horizontal plane PO, though in a direction of rotation R1 opposite to the first direction R and consequently away from the first limit position A.

The restraint means 21 consist essentially in a hinge by which the platform 2 is anchored to the first horizontal axis or pivot X. More exactly, the first axis X occupies a portion of the platform 2 separated from one end 22 (the end supporting the feet of the patient, in the example illustrated) by a distance D2 no greater than the fixed height DX of the selfsame horizontal axis X above datum.

The actuating and positioning means 3 consist in elements of controllably variable length, pivotably associated at one first or stationary end 31 with a second fixed horizontal axis W and at a second or moving end 32 with the platform 2.

The second horizontal axis W is disposed parallel with and lower than the first horizontal axis X, which provides the hinge for the platform 2, and distanced horizontally from the vertical plane occupied by the first axis X.

The second or moving end 32 of the actuating and positioning means 3 is anchored pivotably to a third horizontal axis J associated with the end 25 of the platform 2 opposite from the end 22 nearer to the first horizontal axis X.

Accordingly, by extending or reducing the length of the actuating and positioning means 3, the angle of the platform 2 can be varied selectively between the first limit position A and the second limit position Z.

As indicated in fig 1, the length of the actuating and positioning means 3 is variable between a given minimum value LZ corresponding to the second limit position Z, and a maximum value LA corresponding to the first limit position A. In effect, LA denotes the maximum distance between centres of the second and third horizontal axes W and J, which occurs when the platform 2 occupies a vertical position with the end 22 supporting the lower limbs of the patient at the bottom, whilst the minimum value LZ occurs in a configuration of the platform 2 whereby the lower limbs of the patient (supported by the selfsame first end 22) are elevated in relation to the head (supported by the opposite end 25 of the platform).

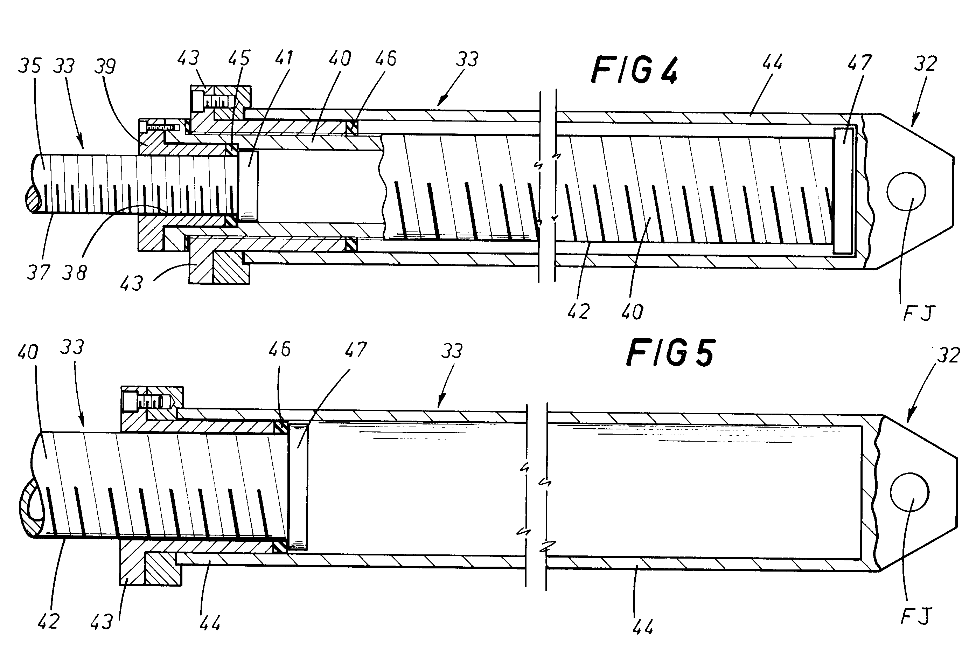

Figs 3, 4 and 5 illustrate a possible embodiment of the actuating and positioning means 3, which are shown in configurations corresponding respectively to the minimum value LZ, to an intermediate value and to the maximum value LA of the distance between centres of the second and third horizontal axes W and J.

In the particular example indicated, the actuating and positioning means 3 consist in a plurality of telescoping elements mutually associated by way of male and female screw threads, denoted 33 in their entirety, which extend between the stationary and moving ends 31 and 32.

The stationary end 31 affords a pineye FW such as will accommodate a pivot coinciding with the second horizontal axis W, and in like manner, the moving end 32 affords a pineye FJ serving to accommodate a pivot coinciding with the third horizontal axis J.

35 denotes a centre shaft anchored permanently to the stationary end 31, which remains at a fixed distance from the second horizontal axis W and is free to rotate about its own longitudinal axis Y, supported in ball bearings 36 or similar elements and driven by a motor 34 (see fig 2) in conjunction with transmission means 12 that could take the form of a belt, a chain, a universal joint or the like.

In the example illustrated, the motor 34 is located above the telescoping elements 33 and associated with the fixed end 31 by way of a bracket 13.

The rotatable centre shaft 35 is fashioned with a first external thread 37, and with a first terminal portion of enlarged diameter, or first boss 41, at the end farthest from the stationary end 31; the first thread 37 is coupled with a matching internal thread 38 afforded by a first bush 39 fitted over the centre shaft 35 and rigidly associated in its turn with a hollow element 40 freely ensheathing and slidable with clearance along the shaft 35. The first bush 39 affords a first locating element 45 consisting in a ring, for example, of diametral proportions such as to disallow the passage of the boss 41 presented by the centre shaft 35.

The hollow element 40 is fashioned with a second external thread 42 of pitch different to that of the first external thread 37, over which to screw a second bush 43 inserted in and secured to a sleeve 44 rigidly associated with the moving end 32.

The second bush 43 affords a corresponding second locating element 46, whilst the hollow element 40 terminates at the end remote from the first bush 39 in a second portion of enlarged diameter, or second boss 47, of which the diametral proportions will be such as to disallow its passage through the second locating element 46.

Dissimilar in pitch, and therefore with different coefficients of friction, the first and second external threads 37 and 42 are interconnected in such a manner as to generate consecutive screwing movements; for the purposes of illustration, it is the first thread 37 which generates the first such movement.

In operation, the effect of activating the motor 34 is to set the centre shaft 35 in rotation about its own longitudinal axis Y, whereupon the interaction between the first external thread 37 and the first bush 39 results in the moving end 32, together with the hollow element 40 and the associated sleeve 44, being distanced from the stationary end 31.

This distancing movement terminates when the first locating element 45 registers in contact with the first boss 41, as illustrated in fig 4.

In this situation, the hollow element 40 and the centre shaft 35 are locked together and must rotate as one, with the result that the second bush 43 is forced to translate along the hollow element 40.

Fig 5 illustrates the limit position obtainable by continuing the rotation to the point at which the second locating element 46 registers against the second boss 47.

Needless to say, the first limit position A of the platform 2 might be timed to occur before extension to the travel limit indicated in fig 5.

The table might also be fitted with travel limit sensors 6 and 7 located respectively to operate as the platform 2 reaches the first limit position A and the second limit position Z.

In effect, the telescoping elements 33 consist in a sleeve 44 associated with one end of the actuating and positioning means, and a plurality of coaxial rods housed internally of the sleeve, which are screw-coupled one with another and furnished with travel limit stops.

To advantage, the movement obtained with actuating and positioning means of the type thus described is essentially linear and easily controlled; moreover, the table can be tilted to an infinitely variable angle and locked in the desired position, thanks to the adoption of positive threaded couplings between the various telescoping elements.

The pivots coinciding with the first and second horizontal axes X and W might be supported by a pedestal 5 comprising at least one plate 4 such as can be anchored to the floor, whether directly, or indirectly by way of a plinth, and exhibiting the shape of a right angle triangle or a portion of a right triangle, having one side of length not less than the fixed height DX aforementioned.

Numerous modifications and variations might be made to the invention, all of which falling within the scope of the basic concept. Moreover, all details may be replaced by alternative elements equivalent in terms of the art.

## Claims

1. A table for diagnostic examinations, of the type comprising a platform (2), anchored pivotably by way of restraint means (21) to a first horizontal axis (X) and rotatable thus through a plurality of positions compassing and including a first limit position (A) in which the platform (2) is disposed substantially vertical, rotated 90^{o} about the first horizontal axis (X) in a first direction (R) away from a horizontal reference plane (PO) occupied by the selfsame first axis (X) and disposed at a first fixed height (DX) above datum, and a second limit position (Z) in which the platform (2) is rotated away from the horizontal reference plane (PO) in a direction (R1) opposite to the first direction,
characterized
- in that the restraint means (21) are associated with a first end (22) of the platform (2), at a point separated from the relative extremity by a distance (D2) no greater than the fixed height (DX) of the first horizontal axis (X) above datum; and
- in that it comprises actuating and positioning means (3) consisting in elements of selectively variable combined length, pivotably associated at a first end or stationary end (31) with a second horizontal axis (W) disposed parallel, beneath and vertically offset relative to the first horizontal axis (X), and at a second end or moving end (32) with a third horizontal axis (J) passing through the platform (2) at the end opposite to the first end (22) occupied by the first horizontal axis (X), in such a way that an increase or a reduction in length of the actuating and positioning means (3), and consequently of the distance between centres of the second and third horizontal axes (W, J), will cause the platform (2) to assume a position coinciding with or between the first and second limit positions (A, Z).

2. A table for diagnostic examinations as in claim 1, wherein the actuating and positioning means (3) comprise a plurality of telescoping elements (33) coaxially insertable through and connected with one another by way of male and female screw threads, and driven by a motor (34) between a configuration of minimum extension (LZ) corresponding to the second limit position (Z) and a configuration of maximum extension (LA) corresponding to the first limit position (A).

3. A table for diagnostic examinations as in claim 2, wherein the actuating and positioning means (3) comprise a sleeve (44) associated with one end (32) and, housed internally of the sleeve, a plurality of coaxial rods screw-coupled one with another, furnished with travel limit stops and designed to generate consecutive screwing movements.

4. A table for diagnostic examinations as in claim 2, wherein the actuating and positioning means (3) comprise a sleeve (44) rigidly associated with the moving end (32), accommodating a centre shaft (35) driven by the motor (34) and a plurality of hollow elements (40, 43) screw-coupled one with another and furnished with respective striking travel limit elements (45, 41, 46, 47).

5. A table for diagnostic examinations as in claim 1, wherein the first and second horizontal axes (X, W) are supported by a pedestal (5) comprising at least one plate anchored to the floor and exhibiting the shape of a right angle triangle or a portion of a right triangle, having one side of length not less than the fixed height (DX) of the first horizontal axis (X) above datum.
